# DEMANDE DE BREVET EUROPEEN

(11) **EP 1 362 615 A1**
(43) Date de publication de la demande: **19.11.2003**
(21) Numéro de dépôt: 03291121.6
(22) Date de dépôt: 14.05.2003
(51) Int. Cl.: A61N 1/372

(54) **Système de téléassistance pour la programmation des dispositifs médicaux implantables actifs tels que stimulateurs cardiaques, défibrillateurs, cardioverteurs ou dispositifs multisite**

(30) Priorité: 17.05.2002 FR 0206072
(71) Demandeur: ELA MEDICAL, 92541 Montrouge (FR)
(72) Inventeur: Ripart, Alain, 91190 Gif sur Yvette (FR); Poulet, Richards, 92120 Montrouge (FR)
(74) Mandataire: Dupuis-Latour, Dominique

(57) **Abrégé**

Un poste de lecture est équipé d'un programmateur pourvu de moyens de lecture par télémétrie et de moyens de télétransmission, sur une ligne téléphonique, des données ainsi lues et/ou de paramètres internes du programmateur. Un poste d'analyse distant comprend des moyens de réception des données envoyés sur la ligne téléphonique et des moyens d'affichage et/ou d'analyse des données transmises. Le programmateur comprend des moyens de recueil d'un signal vocal et des moyens multi-plexeurs, recevant les données et/ou paramètres et le signal vocal recueilli, et délivrant en sortie un signal multiplexé appliqué aux moyens de télétransmission. Le poste d'analyse comprend un circuit de restitution audio et des moyens démultiplexeurs, recevant le signal de la ligne téléphonique et délivrant en sortie les données et/ou paramètres et, d'autre part, ledit signal vocal. Les données et/ou paramètres délivrées par le multiplexeur sont appliqués aux moyens d'affichage et/ou d'analyse des données, et le signal vocal est appliqué au circuit de restitution audio.

## Description

L'invention concerne les "dispositifs médicaux implantables actifs" tels que définis par la Directive 90/385/CE du 20 juin 1990 du Conseil des communautés européennes. Cette définition inclut les stimulateurs cardiaques, défibrillateurs, cardioverteurs et/ou dispositifs multisite, mais aussi les appareils neurologiques, les pompes de diffusion de substances médicales, les implants cochléaires, les capteurs biologiques implantés, etc., ainsi que les dispositifs de mesure de pH ou encore de mesure d'impédance intracorporelle.

Ces dispositifs, par la suite dénommés simplement "implants", comportent une mémoire de données qui peut être lue au moyen d'un programmateur externe par des techniques de télémétrie en elles-mêmes bien connues. Le programmateur est associé à un micro-ordinateur à la disposition du praticien et comportant un écran d'affichage, un clavier pour l'entrée de commandes et la saisie de données, ainsi que divers moyens de mémorisation et de traitement de données.

Le praticien utilisateur du programmateur est un médecin spécialisé, qui peut ainsi accéder aux données et paramètres de fonctionnement stockés dans la mémoire de l'implant, ainsi qu'aux diverses commandes de "programmation", c'est-à-dire les commandes susceptibles de modifier le paramétrage et le fonctionnement du stimulateur.

Le point de départ de l'invention est le constat que, très souvent, le praticien utilisateur du programmateur a besoin d'interroger un expert, notamment pour pouvoir choisir les réglages les plus appropriés du programmateur et/ou de l'implant, besoin qui prend d'autant plus d'importance avec les appareils les plus perfectionnés, qui disposent d'une très grande variété de réglages qu'il n'est pas toujours aisé de choisir et de paramétrer de la manière la plus appropriée à tel ou tel patient.

Pour satisfaire ce besoin particulier, le EP-A-0 856 333 (Ela Medical SpA) propose de mettre en oeuvre une liaison entre le poste de lecture où se trouvent le praticien et le site distant où se trouve l'expert, avec plusieurs lignes téléphoniques, comprenant :
- une ligne pour la transmission des paramètres depuis le stimulateur vers le site distant,
- une deuxième ligne téléphonique, pour l'établissement d'une communication vocale bidirectionnelle entre le praticien et l'expert, et
- une troisième ligne pour la transmission éventuelle, en temps réel, d'un électrocardiogramme de surface.

La pratique montre en effet qu'une liaison vocale est absolument indispensable pour une téléassistance efficace, car la seule transmission de données, même accompagnée de messages sous forme de texte bref, est insuffisante pour obtenir une aide satisfaisante, qui requiert une interactivité totale entre les constatations et les manipulations opérées par le praticien et les conseils prodigués par l'expert.

En pratique cette proposition, qui nécessite deux, voire trois, liaisons téléphoniques distinctes, se révèle difficile à mettre en oeuvre en milieu hospitalier.

En effet, en général les salles d'électrophysiologie ne disposent actuellement que d'une seule ligne téléphonique, ce qui impose soit d'équiper spécialement la salle de deux lignes, soit d'effectuer un branchement, malcommode, sur une deuxième prise située dans une salle voisine.

En raison des contraintes propres à l'environnement hospitalier, il n'est au surplus pas possible d'utiliser pour la liaison vocale un téléphone portable, l'utilisation de ces appareils étant interdite en milieu hospitalier en raison des risques de perturbation des équipements environnants par les signaux électromagnétiques puissants produits par ces téléphones.

L'invention a pour but de résoudre cette difficulté, en proposant un système de téléassistance permettant à l'opérateur d'un programmateur d'entrer en contact avec un expert, d'entretenir une conversation téléphonique permanente avec celui-ci, et de lui transmettre des données et/ou des paramètres de réglage du programmateur sans que cette transmission ne vienne interférer sur la conversation - et ceci en utilisant, sans modification, les infrastructures généralement disponibles dans les salles d'électrophysiologie actuelles.

La télétransmission de données provenant d'un dispositif médical implantable tel qu'un stimulateur cardiaque par l'intermédiaire d'une liaison téléphonique est en elle-même bien connue, par exemple d'après les DE-A-44 01 443 ou US-A-5,586,556, où les données sont numérisées et converties en signaux audio transmis par un combiné téléphonique à un site distant.

Il s'agit cependant d'une situation différente de celle de l'invention : dans ces techniques connues, il ne s'agit pas d'une téléassistance apportée à un praticien pendant que celui-ci utilise un programmateur ; il s'agit seulement d'un système de télésurveillance à la disposition d'un patient isolé, qui permet en outre une reprogrammation éventuelle, à distance, du stimulateur (télémanipulation). En d'autres termes, cette technique est destinée à des patients isolés et elle est utilisable en tous lieux, notamment et surtout en dehors d'un cabinet médical ou d'une salle d'électrophysiologie.

Le système de téléassistance de l'invention est du type général décrit par le EP-A-0 856 333 précité, c'est-à-dire un système comprenant, d'une part, un poste de lecture équipé d'un programmateur et, d'autre part, un poste d'analyse distant, le poste de lecture et le poste d'analyse étant reliés par une liaison téléphonique. Le programmateur du poste de lecture comprend des moyens de lecture par télémétrie d'une mémoire de données du dispositif, et des moyens de télétransmission vers le site distant desdites données lues et/ou de paramètres internes du programmateur, ces moyens de télétransmission étant couplés à ladite ligne téléphonique.

Le poste d'analyse distant comprend des moyens de réception des données envoyés sur la ligne téléphonique par les moyens de télétransmission, et des moyens d'affichage et/ou d'analyse des données ainsi transmises.

Selon l'invention, le programmateur comprend des moyens de recueil et de numérisation d'un signal vocal, et des moyens multiplexeurs, aptes à recevoir en entrée d'une part lesdites données lues et/ou paramètres du programmateur et, d'autre part, le signal vocal recueilli et numérisé, et à délivrer en sortie un signal multiplexé appliqué aux moyens de télétransmission. Quant au poste d'analyse, il comprend un circuit de restitution de signaux audio et des moyens démultiplexeurs, aptes à recevoir en entrée le signal reçu sur la ligne téléphonique et à délivrer en sortie, d'une part, lesdites données lues et/ou paramètres internes et, d'autre part, ledit signal vocal. Les données lues et/ou paramètres internes délivrées par le multiplexeur sont appliqués auxdits moyens d'affichage et/ou d'analyse des données, et le signal vocal est appliqué au circuit de restitution des signaux audio.

L'invention peut être mise en oeuvre en intégrant un circuit multiplexeur à un programmateur préexistant.

On peut en particulier utiliser à cet effet le programmateur *Orchestra* commercialisé par Ela Médical. En effet, cet appareil, outre les moyens de lecture par télémétrie et le modem pour la télétransmission des données, intègre déjà un microphone et une carte son (utilisés à d'autres fins, par exemple pour le pilotage du programmateur par commande vocale).

Le circuit multiplexeur est un circuit d'un type connu permettant de multiplexer des données et de la voix, par exemple semblable aux appareils *AR80* du constructeur Digicom SpA, qui sont des appareils susceptibles de multiplexer ou bien démultiplexer plusieurs canaux sur une même ligne téléphonique, typiquement une ligne RTC, avec au moins un canal de données et un canal vocal. Le fonctionnement du dispositif est symétrique, c'est-à-dire qu'il peut être monté aussi bien en multiplexeur qu'en démultiplexeur, et il gère automatiquement les corrections d'erreurs et l'optimisation de la bande passante. Le multiplexage peut être un multiplexage en mode TDM (division temporelle), SDM (division statistique), ou tout autre mode équivalent ou dérivé de ces derniers.

Il est ainsi possible de transmettre simultanément la voix et les données sur une seule et même ligne téléphonique, et ce de façon entièrement transparente pour les deux utilisateurs, aussi bien le praticien que l'expert.

## Revendications

1. Un système de téléassistance pour la programmation des dispositifs médicaux implantables actifs, notamment les stimulateurs cardiaques, défibrillateurs, cardioverteurs ou dispositifs multisite, ce système comprenant, d'une part, un poste de lecture équipé d'un programmateur et, d'autre part, un poste d'analyse distant, le poste de lecture et le poste d'analyse étant reliés par une liaison téléphonique, le programmateur du poste de lecture comprenant :
- des moyens de lecture par télémétrie d'une mémoire de données du dispositif, et
- des moyens de télétransmission vers le site distant desdites données lues et/ou de paramètres internes du programmateur, ces moyens de télétransmission étant couplés à ladite ligne téléphonique,
et le poste d'analyse distant comprenant :
- des moyens de réception des données envoyés sur la ligne téléphonique par les moyens de télétransmission, et
- des moyens d'affichage et/ou d'analyse des données ainsi transmises, système **caractérisé en ce que** le programmateur comprend :
- des moyens de recueil et de numérisation d'un signal vocal, et
- des moyens multiplexeurs, aptes à recevoir en entrée d'une part lesdites données lues et/ou paramètres du programmateur et, d'autre part, le signal vocal recueilli et numérisé, et à délivrer en sortie un signal multiplexé appliqué aux moyens de télétransmission,
et **en ce que** le poste d'analyse comprend :
- un circuit de restitution de signaux audio, et
- des moyens démultiplexeurs, aptes à recevoir en entrée le signal reçu sur la ligne téléphonique et à délivrer en sortie, d'une part, lesdites données lues et/ou paramètres internes et, d'autre part, ledit signal vocal,
les données lues et/ou paramètres internes délivrées par le multiplexeur étant appliqués auxdits moyens d'affichage et/ou d'analyse des données, et le signal vocal étant appliqué au circuit de restitution des signaux audio.
